# EUROPEAN PATENT SPECIFICATION

(11) **EP 4 112 026 B1**
(45) Date of publication and mention of the grant of the patent: **03.12.2025**
(21) Application number: 22180490.9
(22) Date of filing: 22.06.2022
(51) Int. Cl.: A61F 13/15, B32B 5/02, D04H 1/54, D04H 1/559, D04H 1/593

(54) **COMPOSITE WEB**
VERBUNDSTOFFBAHN
BANDE COMPOSITE

(30) Priority: 30.06.2021 IT 202100017159; 30.06.2021 IT 202100017132
(43) Date of publication of application: 04.01.2023
(73) Proprietor: GDM S.p.A., 40133 Bologna (IT)
(72) Inventor: PIANTONI, Matteo, 40133 Bologna (IT); ZAVALLONI, Alessandro, 40133 Bologna (IT)
(74) Representative: Puggioli, Tommaso

(56) References cited:
- EP-A1- 3 123 993
- EP-A1- 3 431 063
- WO-A1-2014/068488
- US-A- 5 591 148
- US-A1- 2014 023 822
- US-A1- 2015 290 047
- US-A1- 2016 075 122
- US-A1- 2017 079 857
- US-B2- 8 603 277
- US-B2- 9 655 790

## Description

This invention relates to a composite web used to make absorbent sanitary articles, an absorbent sanitary article comprising the composite web, a method for the production of and a unit for forming the composite web.

Generally speaking, absorbent sanitary articles essentially comprise a topsheet, a backsheet and an absorbent core disposed between the topsheet and the backsheet. The topsheet is usually the layer that is in contact with the wearer, while the backsheet is the outer layer of the article.

Known in the prior are absorbent articles, like the one shown, for example, in document WO2016040091A1, which comprise a topsheet comprising what is known as a three-dimensional layer, with bubble-like protrusions obtained by deforming a web using embossing technology; the bubbles allow improving the comfort of the wearer of the absorbent article.

To ensure that the sheet subjected to embossing keeps the desired shape, the topsheet usually also comprises a second layer or base layer joined to the embossed web.

Document US2016075122 discloses a method of forming a composite sheet that is made of a first sheet and a second sheet is disclosed and wherein the first and second sheets are bonded to each other at a number of spaced apart discrete inter-sheet bond locations; the method involves mechanically deforming a precursor nonwoven web to form a first web with deformations therein.

Document EP 3431063 discloses a perforated non-woven fabric formed by overlapping three-dimensional funnel-shaped openings of two perforated non-woven fabric layers and a production method thereof; the first perforated non-woven fabric layer has a protruding portion and a recessed portion and an upper side of the second perforated non-woven fabric layer (is adhered to a lower side of the recessed portion of the first perforated non-woven fabric layer.

Document US8658852 discloses a disposable absorbent articles including a topsheet; a backsheet; a liquid acquisition layer; and a substantially cellulose free absorbent core; wherein the liquid acquisition layer and the absorbent core are located between the topsheet and the backsheet; wherein the topsheet and liquid acquisition layer include corresponding discrete indented regions and unindented regions.

Document EP3123993 discloses a method for producing an absorbent article including: while conveying non-woven fabric to be a top sheet by drawing from the downstream side, forming extruded projections by embossing; placing a material for a second sheet on the back surface of the non-woven fabric with the extruded projections; and joining the non-woven fabric and the material for the second sheet in a joint pattern.

Document WO2014068488 discloses an absorbent article having improved handling of body exudates; the absorbent article can minimize the amount of body exudates in contact with a wearer's skin and can minimize the incidence of leakage of body exudates from the absorbent article.

Document US9655790 discloses an absorbent article which has an absorber disposed between a permeable front surface sheet and a rear surface sheet of a non-woven fabric; wherein the front surface sheet is provided with ridged convex portions continuing in the longitudinal direction of the absorbent article at a specified interval in the width direction of the absorbent article, and provided with dot-like concave embossments at the right side adjacent position and the left side adjacent position alternately at an interval in the longitudinal direction of the absorbent article on the both sides of each of the convex portions, and the concave embossments are arranged in a zigzag pattern as a whole.

Document US2014023822 discloses a shaped sheet laminate that is adapted for an absorbent article and that has a specific structure to enhance the absorbing effect of the absorbent article, a method for manufacturing the shaped sheet laminate, and a shaping apparatus which includes a first roller having a rolling surface, a plurality of indentations that are indented from the rolling surface, a plurality of annular inner wall surfaces respectively defining the indentations, and a plurality of suction holes that are in air communication the respective indentations.

Document US2015290047 discloses methods and apparatuses sensing emitted light caused by fluorescence of adhesive to determine characteristics of absorbent structures during the manufacture of absorbent articles; the inspection system may include a radiation source that illuminates a surface of an absorbent structure with ultraviolet light and a sensor may be adapted to receive light caused by fluorescence of the adhesive while being irradiated with the ultraviolet light.

Disadvantageously, the production of webs for sanitary articles including a topsheet that comprises an unbroken three-dimensional layer applied to a base layer, also unbroken, involves large amounts of material and high production costs.

There is a need to make webs for sanitary articles that are more economical while still ensuring wearer comfort.

This invention therefore has for an aim to meet the above mentioned need.

At least the aim specified is achieved by a composite web, an article, a method for the production of and a unit for forming the composite web in accordance with the independent claims.

The dependent claims correspond to possible different embodiments of the invention.

According to an aspect, the disclosure relates to a composite web, in particular a composite web used for the production of absorbent sanitary articles such as, for example, nappies and the like.

According to an aspect, the composite web comprises a first continuous web that acts as support or matrix.

According to an aspect, the composite web comprises at least one moulded segment. Preferably, the moulded segment is obtained from a second continuous web.

According to an aspect, the moulded segment has at least one protrusion or protuberance and a base portion which at least partly delimits the protrusion itself.

According to an aspect, the moulded portion is joined to the first continuous web by the base portion.

According to an aspect, the first continuous web and the moulded portion delimit at least one cavity at the protrusion.

According to an aspect, the base portion is outside the protrusion.

According to an aspect, in the composite web, in particular in a segment thereof when used in a sanitary article, the protrusions are in contact with the wearer and the first web is in contact with the absorbent core.

In other words, the protrusion protrudes from the base portion towards the wearer; this allows enhancing the comfort of the absorbent article that incorporates the composite web.

According to an aspect, the moulded segment comprises a multiplicity of protrusions delimited by a multiplicity of base portions.

According to an aspect, the first continuous web and the moulded portion delimit a multiplicity of cavities at the protrusions.

According to an aspect, the multiplicity of protrusions defines a motif on the moulded segment.

In other words, the multiplicity of protrusions defines a pattern on the moulded segment.

In an example, the protrusion may have a bubble shape.

In another example, the protrusion may have a wavy shape.

Other examples of shapes that the protrusion may have are the following: circular, diamond, oval, teardrop, elliptical, heart, triangular. Advantageously, the protrusions improve the comfort of the wearer in contact therewith.

According to an aspect, the composite web comprises a multiplicity of moulded segments joined to the first continuous web.

Advantageously, joining the first continuous web to the moulded segment allows fixing the deformation (that is the moulding) imparted to the moulded segment at least at the protrusion.

Advantageously, using moulded segments joined to the first continuous web, which keeps them in shape, allows reducing the amount of material needed to make a comfortable topsheet.

In particular, using moulded segments instead of an entirely moulded continuous web allows reducing the amount of material needed.

Advantageously, reducing the amount of material used allows lowering production costs.

According to an aspect, the moulded segment is moulded using embossing technology.

A continuous web can be cut and spaced (using known *slip and cut* methods) to obtain segments of web which are then moulded by making the segments pass between two rollers provided with male and female portions to obtain moulded segments.

For example, a continuous web can be moulded to obtain an embossed web and this continuous web can then be cut and spaced (for example, using known *slip and cut* methods) so as to obtain moulded segments of first web.

According to an aspect, the moulded segment comprises non-woven fabric.

According to an aspect, the first web comprises non-woven fabric.

By "non-woven fabric" is meant a material whose fibres are randomly oriented.

The first web and/or the moulded segment may comprise different materials such as, for example: carded fibres, non-woven fabrics, cellulose fibres, viscose, polyester, cotton, polyamide, and microfibre. According to an aspect, the composite web comprises at least one weld. Advantageously, the weld allows joining together the base portion of the moulded segment and the first continuous web.

For example, welding may be accomplished by thermal welding or thermomechanical welding or ultrasonic welding.

According to an aspect, the composite web comprises at least one adhesive layer disposed between the base portion of the moulded segment and the first continuous web.

Advantageously, the adhesive layer allows joining together the base portion of the moulded segment and the first continuous web.

According to an aspect, the description regards an absorbent article essentially comprising: a topsheet, an impermeable backsheet and an absorbent core disposed between the topsheet and the backsheet.

According to an aspect, the topsheet comprises a segment of a composite web according to one or more of the aspects set out above.

According to an aspect, the first continuous web is disposed between the moulded segment and the absorbent core.

According to an aspect, an acquisition and distribution Layer (ADL) may be provided between the first continuous web and the absorbent core.

Advantageously, the presence of an ADL allows improving the movement of the liquids which pass through the topsheet to be collected in the absorbent core.

For example, the moulded segment may be made in the form of what is known as a *bubble topsheet*, that is to say, a topsheet with bubbles on it to enhance the comfort of the wearer.

Advantageously, making an absorbent article whose topsheet is made in accordance with one or more of the above-mentioned aspects allows reducing the amount of material needed to produce the absorbent article.

Advantageously, reducing the amount of material needed to make the article allows reducing production costs.

According to an aspect, the first continuous web used for the topsheet joined to the base portion of the moulded segment ensures that the segment keeps the required shape.

Advantageously, ensuring that the protrusions on the moulded segment remain in shape enhances the comfort of the wearer whose skin will be in contact with the protrusions of the sanitary article.

This description relates to a method for the production of composite webs (in particular for making absorbent sanitary articles) according to one or more of the above-mentioned aspects.

According to an aspect, the method comprises a step of feeding a first continuous web.

According to an aspect, the method comprises a step of feeding at least one moulded segment of a second continuous web.

According to an aspect, the moulded segment has at least one protrusion which is at least partly delimited by a base portion.

According to an aspect, the method comprises a step of joining the moulded segment to the first continuous web at the base portion to delimit at least one cavity between the first continuous web and the moulded segment at the protrusion.

Advantageously, joining the first continuous web to the base portion of the moulded segment allows ensuring that the protrusion keeps its shape.

According to an aspect, the method comprises a step of moulding a segment of a second continuous web to obtain the moulded segment.

Advantageously, moulding the segment allows making protrusions that improve the comfort of the wearer of the end product comprising the portion of moulded web.

According to an aspect, the step of moulding is performed using embossing technology.

For example, moulding can basically be performed using a first roller that is peripherally provided with a plurality of suction hollows, and a second roller that is peripherally provided with a plurality of teeth that are substantially shaped to match the suction hollows.

According to an aspect, the method comprises a step of feeding the second continuous web to cut and space it in such a way as to obtain segments of second continuous web.

According to an aspect, the step of cutting and the step of spacing to obtain segments of second web is performed using, for example, the *slip and cut* method.

According to an aspect, the step of joining the moulded segment to the first continuous web comprises a step of welding the moulded segment to the first continuous web at least at the base portion.

Welding may be, for example, thermal, thermomechanical or ultrasonic welding.

According to an aspect, the step of joining the moulded segment to the first continuous web comprises a step of disposing at least one layer of adhesive between the moulded segment and the first continuous web.

According to an aspect, the step of joining the moulded segment to the first continuous web comprises a step of joining a discrete succession of moulded segments to the first continuous web.

Advantageously, joining the moulded segments to the first continuous web allows fixing and keeping the 3D pattern, defined by the protrusions, imparted to the segments.

Advantageously, keeping the 3D pattern moulded on the moulded segments ensures enhanced comfort for the wearer, whose skin will be in contact with the protrusions.

Advantageously, joining moulded segments instead of a continuous moulded web allows reducing the amount of material used for the production of the sanitary article.

Advantageously, using a smaller amount of material allows reducing production costs.

This description relates to a forming unit for making composite webs according to one or more of the above mentioned aspects.

According to an aspect, the forming unit comprises an applicator device configured to join a moulded segment to a first continuous web.

According to an aspect, the moulded segment has a pattern which comprises at least one protrusion and one base portion.

According to an aspect, the forming unit comprises a production device for making the moulded segment.

According to an aspect, the production device comprises a moulding device for moulding the pattern on the segment and a cutting and spacing device for cutting and spacing a second continuous web to make at least one segment or a plurality of segments of second continuous web.

For example, the moulding device may be disposed downstream of the cutting and spacing device in a feed direction of the second continuous web so as to mould the pattern on the segment or segments of the plurality of segments.

In an example, the moulding device may be disposed upstream of the cutting and spacing device in a feed direction of the second continuous web so as to mould the pattern on the second continuous web.

The moulding device is, for example, an embossing device.

According to an aspect, the moulding device comprises a first drum and a second drum positioned relative to each other to define a gap through which the segment of second web or the second web can pass.

Advantageously, the moulding device allows making the three-dimensional pattern, made up of the protrusions on the moulded segment, thereby enhancing the comfort of the wearer, whose skin will be in contact with the moulded segment of the sanitary article being worn.

According to an aspect, the segments of second web or the second web are moulded by conveying them between the drums of the moulding device.

According to an aspect, the axes of the drums of the moulding device are parallel to each other.

According to an aspect, the first drum is rotatable about a respective axis, for example, anticlockwise, and the second drum is rotatable about a respective axis, for example, clockwise (or vice versa).

According to an aspect, the applicator device is located downstream of the production device in a feed direction of the moulded segment.

According to an aspect, the applicator device is configured to join the moulded segment to the first continuous web at the base portion.

According to an aspect, the cutting and spacing device comprises an anvil and a rotary knife acting in conjunction with the anvil.

According to an aspect, the cutting and spacing device, in particular the anvil, is configured to space the segments of second web.

According to an aspect, the segments are cut and spaced, for example, using a slip and cut method on the anvil itself.

According to an aspect, the spaced segments continue along their feed path and pass through the gap of the moulding device so as to obtain moulded segments of second web.

According to an aspect, the applicator device comprises at least one applicator drum tangent to the first drum or to the second drum.

According to an aspect, the applicator device comprises a welder for welding the moulded segment to the first continuous web by thermal or thermomechanical welding.

For example, welding is ultrasonic welding.

According to an aspect, the applicator device comprises an adhesive dispenser, the adhesive dispenser being located downstream of the production device in the feed direction of the moulded segment.

Advantageously, the applicator device allows keeping the shape that the moulding device has imparted to the moulded segment.

According to an aspect, the first drum and the second drum of the moulding device are provided, respectively, with male portions and female portions, which are shaped to match each other and configured to mould the 3D pattern (defined by the protrusions) on the moulded segment when the web passes through the gap.

The male and female portions are shaped according to the shape which the protrusions are intended to have.

The main features of the invention are more apparent from the detailed description which follows, with reference to the accompanying drawings which illustrate a preferred, non-limiting embodiment of the invention, and in which:
- Figure 1 illustrates a detail of a composite web according to the description in a schematic cross sectional view;
- Figure 2 illustrates a detail of a composite web according to the disclosure in a schematic perspective view;
- Figure 3 illustrates a detail of a composite web according to the disclosure in a schematic perspective view;
- Figure 4 illustrates an absorbent article according to the description in a schematic plan view;
- Figure 5 illustrates a unit for forming a composite web according to the description in a schematic front view;
- Figure 6 illustrates a detail of a unit for forming a composite web according to the description in a schematic view;
- Figure 7 illustrates a detail of a unit for forming a composite web according to the description in a schematic view;
- Figure 8 illustrates an absorbent article according to the description in a schematic cross sectional view.

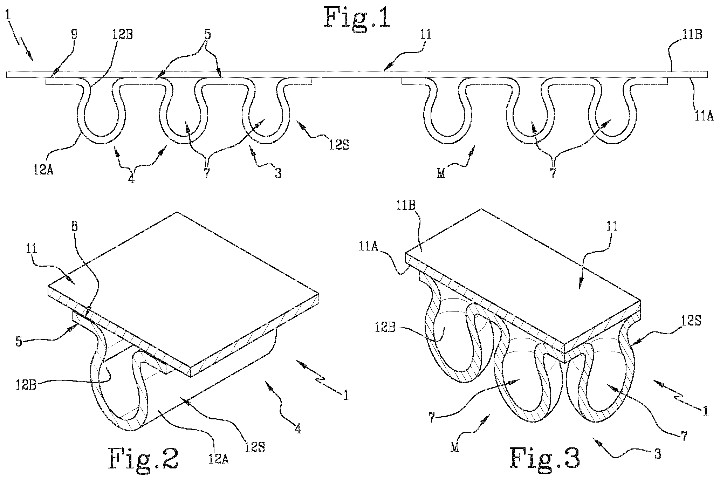

With reference to the accompanying drawings and, in particular, Figure 1, the numeral 1 denotes a composite web according to the description.

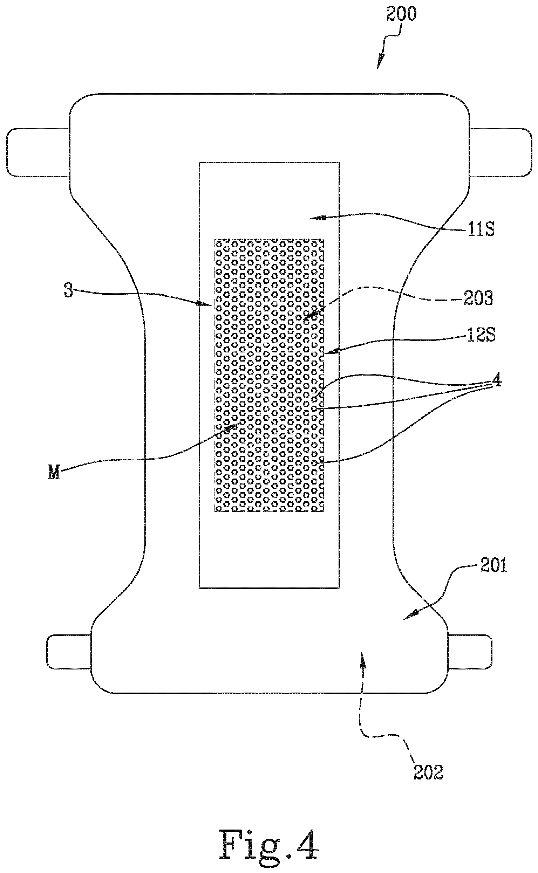

The composite web 1 is preferably intended for the production of absorbent sanitary articles such as nappies, an example of which, labelled 200, is illustrated in Figure 4.

The composite web 1, as explained in more detail below, allows making what are known as three-dimensional or 3D topsheets for nappies.

The composite web 1 comprises a first continuous web 11 and a moulded segment 12S, that is to say, a segment with a pattern M stamped on it.

The moulded segment 12S is obtained from a segment 12L of a second continuous web 12. The segment 12S has a first face 12A and a second face 12B.

Basically, the moulded segment 12S has at least one protrusion 4 which is at least partly delimited by a base portion 5, outside the protrusion 4.

The protrusion 4 protrudes from the first face 12A relative to the base portion 5.

With reference to the accompanying drawings, the cross section of the composite web 1 is greatly scaled up in the illustrations so as to make it easier to understand this description.

The moulded segment 12S defines what is known as the 3D part of the topsheet, made to improve the comfort of the nappy, thanks to the protrusions 4.

The segment 12S thus defines a moulded portion 3 for the composite web 1.

The first continuous web 11 has a first face 11A and a second face 11B.

The first face 11A is joined to the second face 12B of the segment 12S by the base portions 5.

The second face 11B is in contact preferably with the absorbent core 203 of the absorbent sanitary product 200 or with an ADL, if any.

The composite web 1 has at least one cavity 7 which is delimited by the moulded segment 12S and by the web 11 at the protrusions 4.

In other words, the cavity 7 is delimited by the second face 12B of the segment 12S at the protrusion 4 and by the first face 11A of the first continuous web 11.

In an embodiment, the segment 12S, on its first face 12A, has a multiplicity of protrusions 4, delimited by a respective multiplicity of base portions 5 surrounding the protrusions 4.

In this embodiment, the composite web 1 has a multiplicity of cavities 7 delimited by the segment 12S and by the first continuous web 11 (at the multiplicity of protrusions 4).

The multiplicity of protrusions 4 defines the motif M, that is, a pattern that will constitute the three-dimensional portion of the topsheet.

The wearer, when wearing an absorbent sanitary product 200 that comprises the composite web 1, is in contact with the protrusions present on the first face 12A of the segment 12S.

Advantageously, the protrusions 4 improve the comfort for the wearer who is wearing the product and comes into contact with the protrusions.

In an embodiment, the moulded segment 12S comprises non-woven fabric.

In an embodiment, the moulded segment 12S comprises polymer fibres.

In an embodiment, the first continuous web 11 comprises non-woven fabric.

In an embodiment, the first continuous web 11 is a liquid acquisition and distribution layer (ADL).

The shape imparted to the segment 12S by moulding is maintained by gluing the segment 12S to the first continuous web 11.

In an embodiment, the composite web 1 comprises a discrete succession of segments 12S joined to the first web 11.

The composite web 1 has a succession of moulded portions 3 (at the segments 12S), kept in the required shape by the first continuous web 11.

In an absorbent article 200 that uses a segment of the web 1 as topsheet, the segment will have a moulded portion 3, enhancing the comfort of the wearer, at the moulded segment 12S.

In an embodiment, the protrusions 4 of the segment 12S have a bubble shape, illustrated, for example, in Figure 3.

In an embodiment, the protrusions 4 of the segment 12S have a wavy shape, illustrated, for example, in Figure 2.

In an embodiment, the waves moulded on the segment 12S comprise waves with crests 4A that are parallel to the main direction of extension of the segment 12S.

In an embodiment, the waves moulded on the segment 12S comprise waves with crests 4A that are transverse to the main direction of extension of the segment 12S.

The protrusions 4 may have different shapes: for example, diamond, heart, oval, teardrop, elliptic, shamrock and others.

The segments 12S and the first web 11 are joined, for example, by a weld 9, shown in Figure 1, and/or by a layer of adhesive 8, illustrated in Figure 2.

In an embodiment, the segments 12S and the web 11 are joined by welding, in particular thermal welding.

In an embodiment, the segments 12S and the web 11 are joined by ultrasonic welding.

The layer of adhesive 8 is disposed between the segment 12S and the continuous web 11, preferably between the base portions 5 that delimit the protrusions 4 and the web 11.

This description relates to an absorbent article illustrated, for example, in Figure 4 and denoted by the reference numeral 200.

The absorbent article 200, described only insofar as necessary for understanding this invention, essentially comprises: a topsheet 201, an impermeable backsheet 202 and an absorbent core 203 disposed between the topsheet 201 and the backsheet 202.

The topsheet 201 is the part of the article 200 which, in use, is in contact with the wearer.

The topsheet 201 comprises a segment of a composite web 1 according to one or more of the features described above.

The composite web 1 is cut to make a segment of composite web to be assembled in the article 200.

The segment of composite web comprises at least one moulded segment 12S and one segment 11S of the first continuous web 11.

In an embodiment, the moulded segment 12S is smaller in length than the segment 11S.

In an embodiment, the moulded segment 12S is smaller in width than the segment 11S.

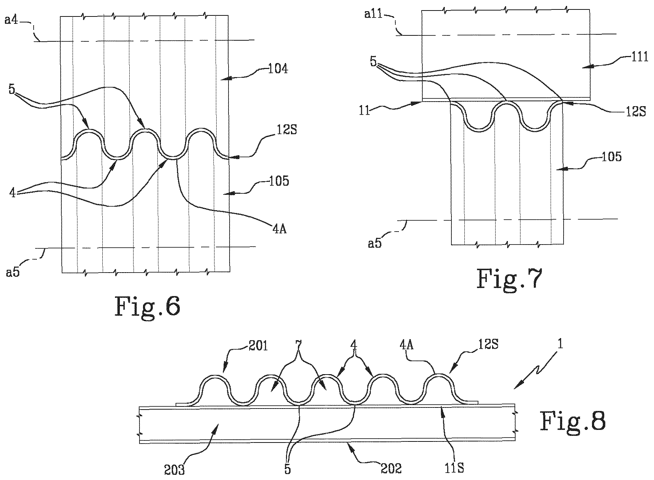

As illustrated, for example, in Figure 8, the segment 11S included in the composite web 1, is disposed between the moulded segment 12S (also included in the composite web 1) and the absorbent core 203.

In an embodiment, not illustrated, the absorbent article 200 comprises an ADL (that is, a liquid acquisition and distribution layer) disposed between the segment 11S and the absorbent core 203. Preferably, the ADL is discrete and disposed at the moulded segment 12S.

Advantageously, the presence of an ADL allows acquiring the liquids and directing them towards an absorbent core, thereby increasing the efficiency of the absorbent sanitary product 200.

This description relates to a method for the production of a composite web 1. The composite web 1 is preferably used for making absorbent sanitary articles such as, for example, the article 200.

The method essentially comprises the following steps:
- feeding a first continuous web 11,
- feeding at least one moulded segment 12S of a second continuous web 12, where the segment 12S has at least one protrusion 4 delimited at least partly by a base portion 5,
- joining the segment 12S to the first web 11 at the base portion 5 to delimit at least one cavity 7 between the web 11 and the segment 12S at the protrusions 4.

In an embodiment, the method comprises a step of moulding a segment 12L of second continuous web 12 to obtain the moulded segment 12S.

Preferably, the method, in particular the step of moulding, comprises a step of embossing the segment 12L, that is, stamping or moulding a 3D pattern on it.

The method used to mould the segment is commonly known as *embossing.*

For example, the step of moulding can basically be performed using a first roller that is peripherally provided with a plurality of suction hollows, and a second roller that is peripherally provided with a plurality of teeth that are substantially shaped to match the suction hollows.

In an embodiment, the method comprises the step of feeding the second continuous web 12.

In an embodiment the method comprises a step of cutting and spacing the second continuous web 12 to obtain segments 12L of second web 12.

The segments 12L are subsequently moulded to obtain segments 12S provided with protrusions 4 delimited by base portions 5.

The step of cutting and spacing can be carried out, for example, using a method known as *slip and cut,* that is to say, a method of cutting and sliding whereby (discrete) segments are cut from the web and then spaced apart.

In an embodiment, the step of joining at least one moulded segment 12S to the first continuous web 11 comprises a step of welding the segments 12S to the web 11 at the base portion 5.

In an embodiment, the step of welding the segments 12S to the web 11 is carried out by ultrasonic welding or thermomechanical welding.

In an embodiment, the step of joining the segments 12S to the continuous web 11 comprises a step of disposing a layer of adhesive 8 between the segments 12S and the web 11.

Joining the segments 12S to the web 11 allows fixing and keeping the 3D pattern imparted to the segment 12S by moulding.

In an embodiment, a succession of suitably sized and spaced segments 12S is applied to the web 11.

In an embodiment, the step of joining the segment 12S to the web 11 comprises a step of joining a discrete succession of segments 12S to the first continuous web 11.

The first continuous web 11 is joined at the base portions 5 of the moulded segment 12S, in particular, it is joined on the side opposite the protrusions 4, defining the moulded portion 3 for the composite web 1.

In an embodiment, the method comprises the steps of moulding the continuous web 12 and cutting and spacing the continuous web 12 to obtain segments 12S.

This description relates to a forming unit 100 for making composite webs, for example of the type like the composite web 1.

The unit 100 essentially comprises a production device 101 and an applicator device 110.

The applicator device 110 is configured to join a moulded segment 12S, provided with a pattern M that comprises at least one protrusion 4 and one base portion 5, to a first continuous web 11.

The production device 101 for making the segment 12S comprises a moulding device 102 for moulding the pattern M and a cutting and spacing device 103 configured to cut and space a second continuous web 12 to make at least one segment or a plurality of segments 12L of the second continuous web 12.

The device 102 is of a type known in the trade and is also called *embossing device.*

In an embodiment, the device 102 is disposed downstream of the device 103 in a feed direction V12 of the second web 12 so as to mould the pattern M on the segment 12L.

In an embodiment not illustrated, the device 102 is disposed upstream of the device 103 in the feed direction V12 to mould the pattern M on the web 12. That way, the segments 12S that are cut and spaced are already moulded.

The device 102 comprises a first drum 104 and a second drum 105 positioned relative to each other to define a through-gap 106 for passage of the segment 12L or of the web 12.

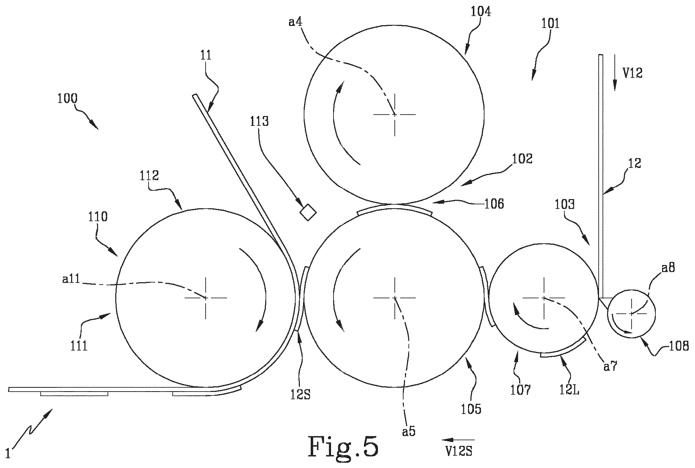

In the example illustrated in Figure 5, the first drum 104 is rotatable clockwise about an axis a4, while the second drum 105 is rotatable anticlockwise about an axis a5, parallel to a4. For simplicity, the drums 104 and 105 are considered to be substantially tangent.

In an embodiment, the first drum 104 and the second drum 105 are provided, respectively, with the male portions and the female portions, which are shaped to match each other and to mould the pattern M (formed of the protrusions 4).

The male - female portions present on the drums 104 and 105 allow making the protrusions 4 on the segment 12S.

The male and female portions are shaped according to the shape which the protrusion 4 is intended to have.

In an embodiment, the segment 12S is thus moulded by conveying it between the drums 104, 105.

The drums 104, 105 shown in Figure 6, for example, are shaped to make wavy protrusions 4.

In an embodiment, the drum 104 is patterned and the drum 105 acts as anvil.

The segment 12L advances and passes through the gap 106 so that the outside surfaces of the drums 104, 105 act in conjunction to engage the segment 12L to impart the 3D pattern to it.

The device 110 is configured to join the segment 12S to the web 11 at the base portion 5.

In an embodiment, the device 110 is located downstream of the production device 101 in a feed direction V12S of the moulded segment.

In an embodiment, as illustrated in the example of Figure 5, the device 103 comprises an anvil 107, preferably in the form of a drum that is rotatable clockwise about an axis a7, and a rotary knife 108 that is rotatable anticlockwise about an axis a8, and acts in conjunction with the anvil 107.

The knife 108 cuts the continuous web 12 to form the segments which are suitably spaced, for example on the anvil 107 itself with a known slip and cut method not further described.

In an embodiment, the device 103, in particular the anvil 107, is configured to space the segments 12L.

The device 110 comprises at least one applicator drum 111 that is tangent to the first drum 104 or to the second drum 105.

The applicator drum 111 is rotatable about an axis a11 clockwise in the example illustrated in Figure 5 and is located downstream of the drum 105 in the feed direction V12S.

In an embodiment, the drum 111 is tangent to the drum 105 and allows carrying out the step of joining the segments 12S to the web 11, that is to say, joining the web 11 to the base portions 5 of the segments 12S, as illustrated schematically in Figure 7.

In an embodiment, the device 110 comprises a welder 112 for welding the moulded segment 12S to the first continuous web 11.

In an embodiment, the drum 112 and the drum 105 are configured to weld the segments 12S to the first web 11.

In an embodiment, the device 110 comprises an adhesive dispenser 113 located downstream of the production device 101 in a feed direction V12S of the moulded segment. The dispenser 113 dispenses at least one layer of adhesive 8 so that the segments 12S can be joined to the continuous web 11 by the adhesive. For example, the dispenser 113 dispenses the adhesive to the web 11 and/or to the segments 12S.

## Claims

1. A composite web (1) comprising:
- a first continuous web (11),
- at least one moulded segment (12S) having at least one protrusion (4) and a base portion (5) at least partly delimiting the protrusion (4), the moulded segment (12S) being obtained from a second continuous web (12); the moulded segment (12S) being joined to the first continuous web (11) by the base portion (5), the first continuous web (11) and the moulded segment (12S) delimiting at least one cavity (7) at the protrusion (4)
the composite web (1), comprising a multiplicity of moulded segments (12S), the moulded segments (12S) being joined to the first continuous web (11).

2. The composite web (1) according to claim 1, wherein the moulded segment (12S) comprises a multiplicity of protrusions (4), the protrusions (4) being delimited by a multiplicity of base portions (5), the moulded segment (12S) being joined to the first continuous web (11) by the base portions (5), the first continuous web (11) and the moulded segment (12S) delimiting a multiplicity of cavities (7) at the protrusions (4).

3. The composite web (1) according to claim 2, wherein the multiplicity of protrusions (4) defines a pattern (M) on the moulded segment (12S).

4. The composite web (1) according to any one of the preceding claims, wherein the moulded segment (12S) comprises a protrusion (4) having a bubble shape.

5. The composite web (1) according to any one of the preceding claims, wherein the moulded segment (12S) comprises a protrusion (4) having a wave shape.

6. The composite web (1) according to any one of the preceding claims, comprising at least one weld (9), preferably a thermal weld or a thermomechanical weld or an ultrasonic weld, the base portion (5) and the first continuous web (11) being joined by the weld (9).

7. The composite web (1) according to any one of the preceding claims, comprising at least one adhesive layer (8), the adhesive layer (8) being disposed between the base portion (5) of the moulded segment (12S) and the first continuous web (11).

8. The composite (1) according to any one of the preceding claims, wherein the base portion (5) is outside the protrusion (4).

9. An absorbent article (200) comprising:
- a topsheet (201),
- an impermeable backsheet (202),
- an absorbent core (203) disposed between the topsheet (201) and the backsheet (202), wherein the topsheet comprises a segment of a composite web (1) according to any one of the preceding claims, the first continuous web (11) being disposed between the moulded segment (12S) and the absorbent core (203).

10. A method for the production of a composite web (1) according to any of the preceding claims 1-8, specifically for making absorbent sanitary articles, comprising the following steps:
- feeding a first continuous web (11),
- feeding at least one moulded segment (12S) of a second continuous web (12), the moulded segment (12S) having at least one protrusion (4) delimited at least partly by a base portion (5),
- joining the moulded segment (12S) to the first continuous web (11) at the base portion (5) to delimit at least one cavity (7) between the first continuous web (11) and the moulded segment (12S) at the protrusion (4)
- feeding the second continuous web (12),
- cutting and spacing the second continuous web (12) to obtain the segment (12L) of the second continuous web,
wherein the step of joining the moulded segment (12S) to the first continuous web (11) comprises a step of joining a discrete succession of moulded segments (12S) to the first continuous web (11).

11. The production method according to claim 10, comprising a step of moulding a segment (12L) of the second continuous web (12) to obtain the moulded segment (12S).

12. The production method according to any one of the claims from 10 to 11, wherein the step of joining the moulded segment (12S) to the first continuous web (11) comprises a step of welding the moulded segment (12S) to the first continuous web (11) at least at the base portion (5).

13. The production method according to any one of claims 10 to 12, wherein the step of joining the moulded segment (12S) to the first continuous web (11) comprises a step of disposing a layer of adhesive (8) between the moulded segment (12S) and the first continuous web (11).

14. A forming unit (100) for making a composite web (1) according to any of claims 1-8, the forming unit (100) comprising:
- an applicator device (110) configured to join a moulded segment (12S), provided with a pattern (M) that comprises at least one protrusion (4), and a base portion (5) to a first continuous web (11);
- a production device (101) for making the moulded segment (12S), the production device (101) comprising a moulding device (102) for moulding the pattern (M) and a cutting and spacing device (103) configured to cut and space a second continuous web (12) to make at least one segment (12L) or a plurality of segments (12L) of the second continuous web (12), wherein the moulding device (102) is disposed downstream of the cutting and spacing device (103) in a feed direction (V12) of the second continuous web (12) in order to mould the pattern (M) on the segment (12L) or wherein the moulding device is disposed upstream of the cutting and spacing device (103) in a feed direction of the second continuous web (12) in order to mould the pattern (M) on the second continuous web (12),
the moulding device (103) comprising a first drum (104) and a second drum (105) positioned relative to each other to define a through-gap (106) for passage of the segment (12L) of second web or of the second continuous web (12), the first drum (104) and the second drum (105) comprising male and female portions, respectively, the male and female portions being shaped to match each other so as to mould the pattern (M) on the moulded segment (12S);
the applicator device (110) being configured to join the moulded segment (12S) to the first continuous web (11) at the base portion (5), the applicator device (110) being disposed downstream of the production device (101) in a feed direction (V12S) of the moulded segment.

15. The forming unit according to claim 14, wherein the cutting and spacing device (103) comprises an anvil (107) and a rotary knife (108) acting in conjunction with the anvil (107).

16. The forming unit according to claim 14 or 15, wherein the cutting and spacing device (103), specifically the anvil (107), is configured to space the segments (12L) of second web (12).

17. The forming unit according to any one of claims 14 to 16, wherein the applicator device (110) comprises at least one applicator drum (111) that is tangent to the first drum (104) or to the second drum (105).

18. The forming unit according to any one of claims 14 to 17, wherein the applicator device (110) comprises a welder (112) for welding the moulded segment (12S) to the first continuous web (11).

19. The forming unit according to any one of claims 14 to 18, wherein the applicator device (110) comprises an adhesive dispenser (113), the adhesive dispenser (113) being disposed downstream of the production device (101) in the feed direction (V12S) of the moulded segment.

## Patentansprüche

1. Verbundstoffbahn (1), umfassend:
- eine erste Endlosbahn (11),
- zumindest ein ausgeformtes Segment (12S) mit zumindest einem Vorsprung (4) und einem den Vorsprung (4) zumindest teilweise begrenzenden Basisabschnitt (5), wobei das ausgeformte Segment (12S) aus einer zweiten Endlosbahn (12) erhalten wird; wobei das ausgeformte Segment (12S) durch den Basisabschnitt (5) mit der ersten Endlosbahn (11) zusammengefügt ist, wobei die erste Endlosbahn (11) und das ausgeformte Segment (12S) zumindest einen Hohlraum (7) am Vorsprung (4) begrenzen,
wobei die Verbundstoffbahn (1) eine Vielzahl von ausgeformten Segmenten (12S) umfasst, wobei die ausgeformten Segmenten (12S) mit der ersten Endlosbahn (11) zusammengefügt sind.

2. Verbundstoffbahn (1) nach Anspruch 1, wobei das ausgeformte Segment (12S) eine Vielzahl von Vorsprüngen (4) umfasst, wobei die Vorsprünge (4) durch eine Vielzahl von Basisabschnitten (5) begrenzt sind, wobei das ausgeformte Segment (12S) durch die Basisabschnitte (5) mit der ersten Endlosbahn (11) zusammengefügt ist, wobei die erste Endlosbahn (11) und das ausgeformte Segment (12S) eine Vielzahl von Hohlräumen (7) an den Vorsprüngen (4) begrenzen.

3. Verbundstoffbahn (1) nach Anspruch 2, wobei die Vielzahl von Vorsprüngen (4) ein Muster (M) auf dem ausgeformten Segment (12S) definiert.

4. Verbundstoffbahn (1) nach einem der vorhergehenden Ansprüche, wobei das ausgeformte Segment (12S) einen Vorsprung (4) mit einer Blasenform umfasst.

5. Verbundstoffbahn (1) nach einem der vorhergehenden Ansprüche, wobei das ausgeformte Segment (12S) einen Vorsprung (4) mit einer Wellenform umfasst.

6. Verbundstoffbahn (1) nach einem der vorhergehenden Ansprüche, umfassend mindestens eine Schweißnaht (9), vorzugsweise eine thermische Schweißnaht oder eine thermomechanische Schweißnaht oder eine Ultraschallschweißnaht, wobei der Basisabschnitt (5) und die erste Endlosbahn (11) durch die Schweißnaht (9) zusammengefügt sind.

7. Verbundstoffbahn (1) nach einem der vorhergehenden Ansprüche, umfassend mindestens eine Klebstoffschicht (8), wobei die Klebstoffschicht (8) zwischen dem Basisabschnitt (5) des ausgeformten Segments (12S) und der ersten Endlosbahn (11) angeordnet ist.

8. Verbundstoffbahn (1) nach einem der vorhergehenden Ansprüche, wobei sich der Basisabschnitt (5) außerhalb des Vorsprungs (4) befindet.

9. Absorbierender Artikel (200), umfassend:
- eine Decklage (201),
- eine undurchlässige Unterlage (202),
- einen absorbierenden Kern (203), der zwischen der Decklage (201) und der Unterlage (202) angeordnet ist, wobei die Decklage ein Segment einer Verbundstoffbahn (1) nach einem der vorhergehenden Ansprüche umfasst, wobei die erste Endlosbahn (11) zwischen dem ausgeformten Segment (12S) und dem absorbierenden Kern (203) angeordnet ist.

10. Verfahren zum Produzieren einer Verbundstoffbahn (1) nach einem der vorhergehenden Ansprüche 1-8, insbesondere zum Herstellen von absorbierenden Hygieneartikeln, umfassend die folgenden Schritte:
- Zuführen einer ersten Endlosbahn (11),
- Zuführen mindestens eines ausgeformten Segments (12S) einer zweiten Endlosbahn (12), wobei das ausgeformte Segment (12S) mindestens einen Vorsprung (4) aufweist, der zumindest teilweise durch einen Basisabschnitt (5) begrenzt ist,
- Zusammenfügen des ausgeformten Segments (12S) mit der ersten Endlosbahn (11) am Basisabschnitt (5), um mindestens einen Hohlraum (7) zwischen der ersten Endlosbahn (11) und dem ausgeformten Segment (12S) am Vorsprung (4) zu begrenzen,
- Zuführen der zweiten Endlosbahn (12),
- Schneiden und Beabstanden der zweiten Endlosbahn (12), um das Segment (12L) der zweiten Endlosbahn zu erhalten, wobei der Schritt zum Zusammenfügen des ausgeformten Segments (12S) mit der ersten Endlosbahn (11) einen Schritt zum Zusammenfügen einer diskreten Abfolge von ausgeformten Segmenten (12S) mit der ersten Endlosbahn (11) umfasst.

11. Produktionsverfahren nach Anspruch 10, umfassend einen Schritt zum Ausformen eines Segments (12L) der zweiten Endlosbahn (12), um das ausgeformte Segment (12S) zu erhalten.

12. Produktionsverfahren nach einem der Ansprüche 10 bis 11, wobei der Schritt zum Zusammenfügen des ausgeformten Segments (12S) mit der ersten Endlosbahn (11) einen Schritt zum Schweißen des ausgeformten Segments (12S) mit der ersten Endlosbahn (11) zumindest am Basisabschnitt (5) umfasst.

13. Produktionsverfahren nach einem der Ansprüche 10 bis 12, wobei der Schritt zum Zusammenfügen des ausgeformten Segments (12S) mit der ersten Endlosbahn (11) einen Schritt zum Anordnen einer Klebstoffschicht (8) zwischen dem ausgeformten Segment (12S) und der ersten Endlosbahn (11) umfasst.

14. Formungseinheit (100) zum Herstellen einer Verbundstoffbahn (1) nach einem der Ansprüche 1-8, wobei die Formungseinheit (100) umfasst:
- eine Applikatorvorrichtung (110), die dazu ausgelegt ist, ein ausgeformtes Segment (12S), das mit einem Muster (M) versehen ist, das mindestens einen Vorsprung (4) und einen Basisabschnitt (5) umfasst, mit einer ersten Endlosbahn (11) zusammenzufügen;
- eine Produktionsvorrichtung (101) zum Herstellen des ausgeformten Segments (12S), wobei die Produktionsvorrichtung (101) eine Formvorrichtung (102) zum Ausformen des Musters (M) und eine Schneid- und Abstandsvorrichtung (103) umfasst, die ausgelegt ist, um eine zweite Endlosbahn (12) zu schneiden und zu beabstanden, um mindestens ein Segment (12L) oder eine Vielzahl von Segmenten (12L) der zweiten Endlosbahn (12) herzustellen, wobei die Formvorrichtung (102) stromabwärts der Schneid- und Abstandsvorrichtung (103) in einer Zuführrichtung (V12) der zweite Endlosbahn (12) angeordnet ist, um das Muster (M) auf dem Segment (12L) zu formen oder wobei die Formvorrichtung stromaufwärts der Schneid- und Abstandsvorrichtung (103) in einer Zuführrichtung der zweiten Endlosbahn (12) angeordnet ist, um das Muster (M) auf der zweiten Endlosbahn (12) auszuformen,
wobei die Formvorrichtung (103) eine erste Trommel (104) und eine zweite Trommel (105) umfasst, die relativ zueinander positioniert sind, um einen Durchgangsspalt (106) für den Durchgang des Segments (12L) der zweiten Bahn oder der zweiten Endlosbahn (12) zu definieren, wobei die erste Trommel (104) und die zweite Trommel (105) männliche bzw. weibliche Abschnitte umfassen, wobei die männlichen und weiblichen Abschnitte so geformt sind, dass sie zueinander passen, um das Muster (M) auf dem ausgeformten Segment (12S) auszuformen;
wobei die Applikatorvorrichtung (110) ausgelegt ist, um das ausgeformte Segment (12S) mit der ersten Endlosbahn (11) am Basisabschnitt (5) zusammenzufügen, wobei die Applikatorvorrichtung (110) stromabwärts der Produktionsvorrichtung (101) in einer Zuführrichtung (V12S) des ausgeformten Segments angeordnet ist.

15. Formungseinheit nach Anspruch 14, wobei die Schneid- und Abstandsvorrichtung (103) einen Amboss (107) und ein mit dem Amboss (107) zusammenwirkendes Rotationsmesser (108) umfasst.

16. Formungseinheit nach Anspruch 14 oder 15, wobei die Schneid- und Abstandsvorrichtung (103), insbesondere der Amboss (107), ausgelegt ist, um die Segmente (12L) der zweiten Bahn (12) zu beabstanden.

17. Formungseinheit nach einem der Ansprüche 14 bis 16, wobei die Applikatorvorrichtung (110) zumindest eine Applikatortrommel (111) umfasst, die die erste Trommel (104) oder die zweite Trommel (105) tangiert.

18. Formungseinheit nach einem der Ansprüche 14 bis 17, wobei die Applikatorvorrichtung (110) eine Schweißeinrichtung (112) zum Verschweißen des ausgeformten Segments (12S) mit der ersten Endlosbahn (11) umfasst.

19. Formungseinheit nach einem der Ansprüche 14 bis 18, wobei die Applikatorvorrichtung (110) einen Klebstoffspender (113) umfasst, wobei der Klebstoffspender (113) stromabwärts der Produktionsvorrichtung (101) in der Zuführrichtung (V12S) des ausgeformten Segments angeordnet ist.

## Revendications

1. Bande composite (1), comprenant :
- une première bande continue (11),
- au moins un segment moulé (12S) comportant au moins une saillie (4) et une partie de base (5) délimitant au moins partiellement la saillie (4), le segment moulé (12S) étant obtenu à partir d'une seconde bande continue (12) ; le segment moulé (12S) étant joint à la première bande continue (11) par la partie de base (5), la première bande continue (11) et le segment moulé (12S) délimitant au moins une cavité (7) en correspondance de la saillie (4),
la bande composite (1) comprenant une multiplicité de segments moulés (12S), les segments moulés (12S) étant joints à la première bande continue (11).

2. Bande composite (1) selon la revendication 1, dans laquelle le segment moulé (12S) comprend une multiplicité de saillies (4), les saillies (4) étant délimitées par une multiplicité de parties de base (5), le segment moulé (12S) étant joint à la première bande continue (11) par les parties de base (5), la première bande continue (11) et le segment moulé (12S) délimitant une multiplicité de cavités (7) en correspondance des saillies (4).

3. Bande composite (1) selon la revendication 2, dans laquelle la multiplicité de saillies (4) définit un motif (M) sur le segment moulé (12S).

4. Bande composite (1) selon l'une quelconque des revendications précédentes, dans laquelle le segment moulé (12S) comprend une saillie (4) ayant une forme de bulle.

5. Bande composite (1) selon l'une quelconque des revendications précédentes, dans laquelle le segment moulé (12S) comprend une saillie (4) ayant une forme ondulée.

6. Bande composite (1) selon l'une quelconque des revendications précédentes, comprenant au moins une soudure (9), de préférence une soudure thermique ou une soudure thermomécanique ou une soudure par ultrasons, la partie de base (5) et la première bande continue (11) étant jointes par la soudure (9).

7. Bande composite (1) selon l'une quelconque des revendications précédentes, comprenant au moins une couche adhésive (8), la couche adhésive (8) étant disposée entre la partie de base (5) du segment moulé (12S) et la première bande continue (11).

8. Bande composite (1) selon l'une quelconque des revendications précédentes, dans laquelle la partie de base (5) se trouve à l'extérieur de la saillie (4).

9. Article absorbant (200), comprenant :
- une feuille supérieure (201),
- une feuille arrière imperméable (202),
- un cœur absorbant (203) disposé entre la feuille supérieure (201) et la feuille arrière (202), dans lequel la feuille supérieure comprend un segment d'une bande composite (1) selon l'une quelconque des revendications précédentes, la première bande continue (11) étant disposée entre le segment moulé (12S) et le cœur absorbant (203).

10. Procédé pour la production d'une bande composite (1) selon l'une quelconque des revendications précédentes 1-8, en particulier pour la fabrication d'articles hygiéniques absorbants, comprenant les étapes suivantes :
- alimenter une première bande continue (11),
- alimenter au moins un segment moulé (12S) d'une seconde bande continue (12), le segment moulé (12S) comportant au moins une saillie (4) délimitée au moins partiellement par une partie de base (5),
- joindre le segment moulé (12S) à la première bande continue (11) en correspondance de la partie de base (5) pour délimiter au moins une cavité (7) entre la première bande continue (11) et le segment moulé (12S) en correspondance de la saillie (4),
- alimenter la seconde bande continue (12),
- découper et espacer la seconde bande continue (12) pour obtenir le segment (12L) de la seconde bande continue,
dans lequel l'étape consistant à joindre le segment moulé (12S) à la première bande continue (11) comprend une étape consistant à joindre une succession distincte de segments moulés (12S) à la première bande continue (11).

11. Procédé de production selon la revendication 10, comprenant une étape consistant à mouler un segment (12L) de la seconde bande continue (12) pour obtenir le segment moulé (12S).

12. Procédé de production selon l'une quelconque des revendications de 10 à 11, dans lequel l'étape consistant à joindre le segment moulé (12S) à la première bande continue (11) comprend une étape consistant à souder le segment moulé (12S) à la première bande continue (11) au moins en correspondance de la partie de base (5).

13. Procédé de production selon l'une quelconque des revendications 10 à 12, dans lequel l'étape consistant à joindre le segment moulé (12S) à la première bande continue (11) comprend une étape consistant à disposer une couche d'adhésif (8) entre le segment moulé (12S) et la première bande continue (11).

14. Unité de formage (100) pour fabriquer une bande composite (1) selon l'une quelconque des revendications 1-8, l'unité de formage (100) comprenant :
- un dispositif applicateur (110) configuré pour joindre un segment moulé (12S), pourvu d'un motif (M) qui comprend au moins une saillie (4), et une partie de base (5) sur une première bande continue (11) ;
- un dispositif de production (101) pour fabriquer le segment moulé (12S), le dispositif de production (101) comprenant un dispositif de moulage (102) pour mouler le motif (M) et un dispositif de découpe et d'espacement (103) configuré pour découper et espacer une seconde bande continue (12) afin de fabriquer au moins un segment (12L) ou une pluralité de segments (12L) de la seconde bande continue (12), dans laquelle le dispositif de moulage (102) est disposé en aval du dispositif de découpe et d'espacement (103) dans une direction d'alimentation (V12) de la seconde bande continue (12) afin de mouler le motif (M) sur le segment (12L) ou dans laquelle le dispositif de moulage est disposé en amont du dispositif de découpe et d'espacement (103) dans une direction d'alimentation de la seconde bande continue (12) afin de mouler le motif (M) sur la seconde bande continue (12),
le dispositif de moulage (103) comprenant un premier tambour (104) et un seconde tambour (105) positionnés l'un par rapport à l'autre pour définir un espace traversant (106) pour le passage du segment (12L) de la seconde bande ou de la seconde bande continue (12), le premier tambour (104) et le seconde tambour (105) comprenant respectivement des parties mâles et femelles, les parties mâles et femelles étant façonnées pour s'accoupler l'une à l'autre de manière à mouler le motif (M) sur le segment moulé (12S) ;
le dispositif applicateur (110) étant configuré pour joindre le segment moulé (12S) à la première bande continue (11) en correspondance de la partie de base (5), le dispositif applicateur (110) étant disposé en aval du dispositif de production (101) dans une direction d'alimentation (V12S) du segment moulé.

15. Unité de formage selon la revendication 14, dans laquelle le dispositif de découpe et d'espacement (103) comprend une enclume (107) et un couteau rotatif (108) agissant conjointement avec l'enclume (107).

16. Unité de formage selon la revendication 14 ou 15, dans laquelle le dispositif de découpe et d'espacement (103), en particulier l'enclume (107), est configuré pour espacer les segments (12L) de la seconde bande (12).

17. Unité de formage selon l'une quelconque des revendications 14 à 16, dans laquelle le dispositif applicateur (110) comprend au moins un tambour applicateur (111) qui est tangent au premier tambour (104) ou au seconde tambour (105).

18. Unité de formage selon l'une quelconque des revendications 14 à 17, dans laquelle le dispositif applicateur (110) comprend une machine à souder (112) pour souder le segment moulé (12S) à la première bande continue (11).

19. Unité de formage selon l'une quelconque des revendications 14 à 18, dans laquelle le dispositif applicateur (110) comprend un distributeur d'adhésif (113), le distributeur d'adhésif (113) étant disposé en aval du dispositif de production (101) dans la direction d'alimentation (V12S) du segment moulé.
